# EUROPEAN PATENT SPECIFICATION

(11) **EP 2 557 423 B1**
(45) Date of publication and mention of the grant of the patent: **16.04.2014**
(21) Application number: 12179233.7
(22) Date of filing: 03.08.2012
(51) Int. Cl.: G01N 33/543, G01N 33/68

(54) **Immunoassay of carboxymethylarginine**
Immuntest auf Carboxylmethylarginin
Dosage immunologique de carboxymethylarginine

(30) Priority: 08.08.2011 JP 2011173106
(43) Date of publication of application: 13.02.2013
(73) Proprietor: ARKRAY, Inc., Kyoto-shi, Kyoto 601-8045 (JP)
(72) Inventor: Togashi, Satoko, Kyoto-shi Kyoto 602-0008 (JP); Yagi, Masayuki, Kyoto-shi Kyoto 602-0008 (JP)
(74) Representative: Golding, Louise Ann

(56) References cited:
- K. MERA WT AL.: "Immunological Detection of N.omega.-(Carboxymethyl)arginine by a Specific Antibody", ANNALS OF THE NEW YORK ACADEMY OF SCIENCES, vol. 1126, 2008, pages 155-157, XP002685841, New York ISSN: 0077-8923, DOI: 10.1196/annals.1433.000
- K. IIJIMA ET AL: "Immunological detection of N. omega.-(carboxymethyl)arginine.", INTERNATIONAL CONGRESS SERIES, vol. 1245, 2002, pages 349-351, XP002685842, Amsterdam ISSN: 0531-5131
- ODANI ET AL.: BIOCHEMICAL AND BIOPHYSICAL RESEARCH COMMUNICATIONS, vol. 285, 2001, pages 1232-1236, XP002685843,

## Description

The present invention relates to an immunoassay of carboxymethylarginine.

AGEs (Advanced Glycation End-products) are substances that are formed in the advanced Maillard reaction, and their involvement in aging and in the development and progression of various diseases is becoming evident. For example, it has been known that if AGEs are formed and accumulate in the skin, they become a cause of problems to the skin such as wrinkles, spots, sagging, and deterioration of the skin resilience.

N^{ω}-carboxymethylarginine (hereinafter referred also to as "CMA") is known as one type of AGEs. It has been shown that CMA is an AGE that is formed specifically in collagen (e.g., Japanese Patent No. 3,889,190). For this reason, a CMA formation inhibitor is proposed for the purpose of inhibiting the formation of CMA in a living body to prevent aging and diseases resulting from the formation of CMA as well as the accumulation of CMA in collagen (WO 2011/004734). Further, since diabetics' blood serum protein has a remarkably higher level of CMA than that of healthy subjects of the same age, CMA shows promise for its potential as a new diagnostic marker for glycation in diabetes (Odani et al., Biochemical and Biophysical Research Communications 285, 1232-1236 (2001)) as well as for its usability as a maker for osteoporosis.

Several hundred substances such as pentosidine, carboxymethyllysine (hereinafter referred to as "CML") and pyrraline had been identified as AGEs before CMA was identified as such, and attempts have been made to use these substances as anti-glycation markers. However, pentosidine and CML are not tissue specific. Thus, even if their blood levels are measured, glycating tissue cannot be determined. In contrast, CMAis specific to connective tissue proteins such as collagen as mentioned above, so that values obtained by measurement of CMA may serve as an indicator of glycation of connective tissue proteins (collagen in particular). Therefore, an antibody that specifically identifies CMA and an immunological reagent for detecting CMA including the antibody are proposed (JP 2002-243732 A).

The present invention relates to a method of measuring carboxymethylarginine in a sample. The method includes treating the sample with a pretreatment agent including urea, and measuring carboxymethylarginine in the treated sample by an immunological method.
FIG. 1 is a graph showing exemplary results of Example 1.
FIG. 2 is a graph showing exemplary results of Comparative Example 1.
FIG. 3 is a graph showing exemplary calibration curves obtained based on the results of Example 1 and Comparative Example 1.

When the measuring reagent disclosed in JP 2002-243732 A, such as the antibody, is used for a sample including a CMA preparation in measuring CMA by an immunological method, adequate sensitivity can be obtained. Similarly Mera et al. (Annals of the New York Academy of Sciences 1126, 155 - 157 (2008)) describes the immunological detection of N^{ω} - (Carboxymethyl)arginine by a specific antibody. However, the present inventors found a new problem associated with this measuring reagent; CMA can be hardly detected if the measuring reagent is used for a biological sample (particularly a blood-derived sample). On the other hand, there are other methods for measuring CMAin biological samples, such as, for example, LC/MS. However, LC/MS requires special equipment, and performing a measurement by this method is extremely complicated. For these reasons, the present invention provides a new method of measuring CMA by which CMA can be measured with ease and with a high degree of precision.

In one or more aspects, the present invention relates to a method of measuring carboxymethylarginine in a sample (hereinafter referred also to as "the measuring method of the present invention"). The method includes treating the sample with a pretreatment agent including urea, and measuring carboxymethylarginine in the treated sample by an immunological method.

According to the present invention, it is possible to measure CMAin a sample, such as a biological sample, with ease and with a high degree of precision.

The present invention is based on the findings that CMA in a biological sample can be measured with ease and with a high degree of precision if CMA is measured by an immunological method after pretreating the biological sample with urea (CO(NH₂)₂).

Why pretreatment of a biological sample with urea allows a measurement of CMA in the biological sample by an immunological method is not clear in detail, but the mechanism can be presumed as follows. That is, CMA is considered to be bonded to proteins such as albumin when it is present in a biological sample, particularly in a blood sample. Therefore, even if an antibody that specifically identifies CMA is added to a biological sample, CMA cannot bond to the antibody, making CMA hardly detectable. It is considered that pretreatment of a biological sample with urea causes structural changes in the proteins bonded to CMA and makes the bond between CMA and the proteins unmaintable, thus resulting in an improvement in the reactivity between CMA and the antibody It is to be noted that the present invention is not limited to this mechanism when being interpreted.

According to the measuring method of the present invention, it is possible to track the amount of CMA formed and accumulated in a living body with ease and with a high degree of precision. Attempts have been made to use CMA as markers for anti-glycation, skin aging and/or osteoporosis. Thus, according to the measuring method of the present invention, since it is possible to track the degree of symptoms of and the possibility of the development of glycation stress, skin aging and/or osteoporosis, the measuring method of the present invention is useful in preventing these symptoms and in the field of anti-aging. Moreover, CMA has been known to be formed and/or to accumulate specifically in collagen. Therefore, on the basis of values obtained by measurement of CMA by the measuring method of the present invention, the degree of aging of collagen and/or the degree of aging of the skin can be monitored, for example. And from these results, the degree of aging of humans can be evaluated.

In one or more aspects of the measuring method of the present invention, a sample to be used is preferably a biological sample. This is because the effect of improving the measuring sensitivity resulting from the pretreatment agent is likely to increase in the case of biological samples. Examples of biological samples include urine-derived, blood-derived and tissue-derived samples. Examples of blood-derived samples include whole blood, blood serum, blood plasma and hemolytic samples. Examples of tissue-derived samples include samples prepared from tissue collected from a living body. Examples of types of tissue include those containing collagen, such as bones, skin and tendon. In particular, blood-derived samples are preferable because the effect of improving the measuring sensitivity resulting from the treatment with the pretreatment agent increases more. Of the blood-derived samples mentioned, blood serum and blood plasma samples are more preferable. A sample collected from a living body may be used as it is or may be diluted before the use. Further, according to the measuring method of the present invention, CMA included in a sample can be measured with ease and with a high degree of precision even if CMA is not isolated and/or purified (CMA preparation).

In one or more aspects of the measuring method of the present invention, as long as the pretreatment agent includes an effective amount of urea as an active ingredient, the pretreatment agent may consist essentially of urea, for example. The term "consist essentially of urea" as used herein means that the pretreatment agent consists only of urea other than unavoidable impurities included in the raw material of the pretreatment agent and/or unavoidable impurities blended into the pretreatment agent in the course of production.

To treat a sample with the pretreatment agent including urea, for example, the sample and the pretreatment agent including urea can be brought into coexistence in a solution. By bringing the sample and urea into coexistence, the reactivity between CMA in the sample and the antibody improves, thus enhancing the measuring sensitivity. The method of bringing the pretreatment agent and the sample into coexistence in a solution and the order of the method are not particularly limited. For example, the pretreatment agent may be added to a sample, or a sample may be added to a solution containing the pretreatment agent.

In the step of treating a sample with the pretreatment agent, there is no particular limitation to the concentration of urea (the concentration of urea in a pretreatment solution containing the pretreatment agent and the sample) at the time of treating the sample, and the concentration is, for example, 0.01 M to 10 M, and preferably 0.01 M to 8 M. There is no particular limitation to the pH of the pretreatment solution, and the pretreatment solution has a pH of, for example, 4.0 to 9.0, preferably 5.0 to 8.0, and more preferably 6.0 to 7.5.

There is no particular limitation to the treatment temperature at which a sample is treated with the pretreatment agent, and the treatment temperature is, for example, 0 to 60°C. When carrying out an immunoassay immediately after the treatment, the treatment temperature is preferably 4 to 45°C, and more preferably 25 to 37°C. There is no particular limitation to the treatment time over which a sample is treated with the pretreatment agent, and the treatment time is, for example, 0.1 minutes to 12 hours. In terms of ease of measurement, the treatment time is preferably 0.1 to 30 minutes, and more preferably 0.1 to 10 minutes.

The term immunological method" as used herein refers to a method of detecting or measuring CMA with use of an antibody, a fragment thereof or an analog thereof specific to CMA. Examples of such methods include, but are not limited to, EIA (enzyme immunoassay), immunochromatography and agglutination methods. The immunological method to be used may be determined appropriately in accordance with the type of sample to be used. When a sample to be used is a blood-derived sample, the agglutination method is preferable in terms of improving the measurement precision. Further, the immunological method to be used may be determined appropriately in accordance with the state of CMA in a sample. A non-competitive method is preferable when there is more than one CMA in a macromolecule, and a competitive method is preferable when CMA is present solely in one molecule.

For example, an antigen-antibody reaction between CMA in a sample and an antibody specifically reactive to CMA (hereinafter referred also to as "anti-CMA antibody") may be used to carry out a measurement by an immunological method. Hereinafter, a measurement by an immunological method will be described by taking the agglutination method as an example. The agglutination method may be competitive or non-competitive. There is no particular limitation to a measurement of CMA in a sample by the non-competitive agglutination method. For example, CMA can be measured by mixing particles sensitized to an anti-CMA antibody (hereinafter referred to as "antibody-sensitized particles") with a sample pretreated in the above-described manner, and measuring the agglutination of the antibody-sensitized particles.

The anti-CMA antibody may be a monoclonal or polyclonal antibody Examples of the anti-CMA antibody include the antibody disclosed in JP 2002-243732 A. A commercially available anti-CMA antibody or self-prepared anti-CMA antibody may be used. There is no particular limitation to particles to be sensitized to an antibody, and known particles generally used in immunological methods can be used. Examples of such particles include insoluble carrier particles and magnetic particles. There is no particular limitation to insoluble carrier particles, and examples of insoluble carrier particles include latex particles such as polystyrene latex particles, polypropylene particles, polyethylene particles, gelatin particles, and colloidal gold particles. In particular, latex particles are preferable. There is no particular limitation to the particle size of the particles. In the case of latex particles, their particle size is, for example, 0.05 to 1.0 µm. In terms of ease of maintenance of the dispersibility and ease of a measurement of the degree of agglutination, the particle size is preferably 0.10 to 0.50 µm. There is no particular limitation to the method of sensitizing particles to an anti-CMA antibody, and known methods in the art may be used. Examples of such methods include physical adsorption and chemical bonding. For example, antibody-sensitized particles may be suspended in a buffer solution or water and used in the form of a suspension. There is no particular limitation to the percentage of the particles in the suspension, and the percentage is, for example, 0.01 to 1 wt%. Examples of buffers usable as the buffer solution include a Good's buffer, a phosphate buffer and a Tris buffer.

There is no particular limitation to the reaction temperature at which a pretreated sample and antibody-sensitized particle are reacted with each other. For example, the reaction temperature is preferably 4 to 45°C, and more preferably 25 to 37°C. There is no particular limitation to the reaction time, and the reaction time is, for example, 0.1 to 60 minutes. In terms of improving the measuring sensitivity and the speed of the measurement, the reaction time is preferably 1 to 30 minutes, and more preferably 3 to 15 minutes. There is no particular limitation to the concentration of antibody-sensitized particles in a mixed solution of the antibody-sensitized particles and a sample, and the concentration is, for example, 0.001 to 0.2 w/v%, and preferably 0.02 to 0.07 w/v%. There is no particular limitation to the measurement of agglutination. The absorbance and/or the turbidity can be measured by a spectrophotometer or optical measuring device to measure the agglutination.

There is no particular limitation to a measurement of CMA in a sample by the competitive agglutination method. For example, CMA can be measured by mixing a CMA carrying substance with a sample pretreated in the above-described manner, mixing the sample mixed with the CMA carrying substance with antibody-sensitized particles, and measuring the agglutination of the antibody-sensitized particles. Examples of CMA carrying substances include particles sensitized to CMA (antigen), those obtained by bonding CMA to carrier substances of hemocyanin and albumin, and glycation collagen. Glycation collagen refers to glycated collagen, and it can be prepared by brining collagen and glucose into coexistence in a neutral phosphate buffer solution and incubating them at 37°C. The method of preparing glycation collagen is disclosed in, for example, JP 2002-243732 A.

There is no particular limitation to the reaction temperature at which the pretreated sample and the CMA carrying substance are reacted with each other. For example, the reaction temperature is preferably 4 to 45°C, and more preferably 25 to 37°C. There is no particular limitation to the reaction time, and the reaction time is, for example, 0 to 60 minutes. In terms of ensuring the speed of the measurement and the uniformity of the solution, the reaction time is preferably 0.5 to 10 minutes, and more preferably 0.5 to 5 minutes. There is no particular limitation to the concentration of the CMA carrying substance in the mixed solution of the sample and the CMA carrying substance, and the concentration is, for example, 0.001 to 0.2 w/v%, and preferably 0.001 to 0.05 w/v%. The reaction temperature and the reaction time at which and over which the sample and the antibody-sensitized particles are reacted with each other and the concentration of the antibody-sensitized particles in the mixed solution are the same as those in the non-competitive agglutination method described above.

In one or more aspects of the measuring method of the present invention, the measuring method may include quantifying CMA in the sample based on results of measuring CMA by the immunological method. By quantifying CMA in the sample, it is possible to track the degree of symptoms of and the possibility of the development of glycation stress, skin aging and/or osteoporosis and to determine and/or monitor the degree of aging of collagen and/or the degree of aging of the skin.

Thus, another aspect of the present invention may relate to a method of determining glycation stress, osteoporosis, the degree of aging of collagen or the degree of aging of the skin. The method includes treating a sample with a pretreatment agent including urea, measuring CMA in the pretreated sample by an immunological method, and quantifying CMA in the sample based on results of measuring CMA by the immunological method.

### (Method of Pretreating Sample)

Still another aspect of the present invention relates to a method of pretreating a sample in measuring CMA in the sample by an immunological method. The method includes treating the sample with a pretreatment agent including urea. By pretreating a sample by the pretreatment method of the present invention, CMA in the sample can be measured by a highly-precise immunological method. Conditions under which a sample is pretreated with the pretreatment agent may be the same as those in the method of measuring CMA described above.

### (Pretreatment Agent)

Still another aspect of the present invention relates to the use of a pretreatment agent for treating a sample in measuring CMA by an immunological method. The pretreatment agent includes urea. According to the pretreatment agent of the present invention, pretreatment of a sample for measuring CMA in the sample by an immunological method can be carried out with ease.

As long as the pretreatment agent includes an effective amount of urea as an active ingredient, the pretreatment agent may consist essentially of urea, for example.

There is no particular limitation to the form of the pretreatment agent, and the pretreatment agent may be of a liquid (liquid reagent) or dry type. However, in terms of ease of use, it is preferable that the pretreatment agent is in the form of a liquid reagent obtained by dissolving the pretreatment agent in a buffer solution or water. When the pretreatment agent is of a liquid type, there is no particular limitation to the concentration of urea in the pretreatment agent, and the concentration is, for example, 0.01 to 10 M, and preferably 0.01 to 8 M.

### (Measuring Kit for Use in Measuring CMA by Immunological Method)

Still another aspect of the present invention relates to a measuring kit for use in measuring CMAby an immunological method. The measuring kit includes a pretreatment agent including urea, and a measuring reagent for use in measuring CMA by an immunological method. According to the measuring kit of the present invention, it is possible to measure CMAby an immunological method with ease. Examples of the pretreatment agent include the pretreatment agent described above.

There is no particular limitation to the measuring reagent. As long as the measuring kit includes a reagent capable of detecting or measuring CMA with use of an antibody, a fragment thereof, or an analogue thereof specific to CMA, the reagent can be configured appropriately in accordance with the immunological method by which a measurement is carried out. In particular, the measuring reagent preferably includes, for example, anti-CMA antibody-sensitized particles. It is possible to use the anti-CMA antibody-sensitized particles disclosed above in connection with the method of measuring CMA.

The measuring kit of the present invention may additionally include, for example, a sample preparation solution for use in diluting a sample. There is no particular limitation to sample preparation solutions, and example of which include a buffer solution and water.

The measuring kit of the present invention preferably includes an instruction manual describing a method of measuring CMA in a sample by an immunological method using the above-described reagent. This may include when an instruction manual is not included in the measuring kit of the present invention but is provided on the web.

The measuring kit of the present invention may be in the form of a reagent pack in which the pretreatment agent and the measuring reagent are contained, for example. For example, the reagent pack preferably is in the form of a disposable cartridge and more preferably includes two or more kinds of vessels in which the reagent can be placed. It is preferable that the measuring kit of the present invention is in the form of a reagent pack in which the pretreatment agent and the measuring reagent are individually placed in vessels. There is no particular limitation to the reagent pack, and the reagent pack may further include, for example, one or more of a reaction vessel, a specimen vessel, a dispensation chip, an optical reaction cell, and a disposal vessel. By using such a reagent pack, the measuring method of the present invention can be carried out more easily and the application of the measuring kit to an automated device becomes extremely easy.

### (Pretreatment Kit)

Still another aspect of the present disclosure relates to a pretreatment kit for use in treating a sample in measuring CMA in the sample by an immunological method. The pretreatment kit includes a pretreatment agent including urea. According to the pretreatment kit of the present disclosure, it is possible to carry out the pretreatment method of the present invention with ease. It is preferable that the pretreatment kit of the present invention additionally includes, for example, a sample preparation solution for use in diluting a sample, and/or an instruction manual describing a method of pretreating a sample using the above-described pretreatment agent.

The present invention may relate to one or more of the following aspects.
[1] A method of measuring carboxymethylarginine in a sample, the method including:
   treating the sample with a pretreatment agent including urea; and
   measuring carboxymethylarginine in the treated sample by an immunological method.
[2] The method according to [1], wherein the pretreatment agent includes urea as an active ingredient.
[3] The method according to [1] or [2], wherein the sample is a biological sample.
[4] The method according to [3], wherein the biological sample is a urine- derived, blood-derived, or tissue-derived sample.
[5] The method according to any one of [1] to [4], wherein the immunological method is selected from the group consisting of enzyme immunoassay, immunochromatography, and agglutination methods.
[6] The method according to any one of [1] to [5], wherein the immunological method comprises mixing the treated sample with antibody-sensitized particles sensitized with an antibody reactive to carboxymethylarginine, and measuring agglutination of the antibody-sensitized particles.
[7] The method according to [6], wherein the antibody-sensitized particles are antibody-sensitized particles obtained by sensitizing insoluble carrier particles to an antibody.
[8] The method according to [7], wherein the insoluble carrier particles are selected from the group consisting of latex particles and colloidal gold particles.
[9] The use of a pretreatment agent for treating a sample in measuring carboxymethylarginine by an immunological method, the pretreatment agent including urea.
[10] The use according to [9], including an effective amount of urea as an active ingredient.
[11] A measuring kit for use in measuring carboxymethylarginine by an immunological method, the measuring kit including:
   a pretreatment agent including urea; and
   a measuring reagent for use in measuring carboxymethylarginine by an immunological method.
[12] The measuring kit according to [11], wherein the measuring reagent includes antibody-sensitized particles.
[13] The measuring kit according to [12], wherein the antibody-sensitized particles are antibody-sensitized particles obtained by sensitizing insoluble carrier particles to an antibody.
[14] The measuring kit according to [13], wherein the insoluble carrier particles are selected from the group consisting of latex particles and colloidal gold particles.
[15] Use of urea for the production of a pretreatment agent for treating a sample in measuring carboxymethylarginine by an immunological method.
[16] Use of urea as a pretreatment agent for treating a sample in measuring carboxymethylarginine by an immunological method.
[17] A method for pretreating a sample in measuring carboxymethylarginine in the sample by an immunological method, the method including treating the sample with a pretreatment agent including urea.

Hereinafter, the present invention will be described in more detail by way of Example 1 and Comparative Example 1. It is to be noted that the present invention is not limited to the following examples when being interpreted.

### EXAMPLES

### [Preparation of Anti-CMA Polyclonal Antibody-Sensitized Latex Particles]

Anti-CMA polyclonal antibody-sensitized latex particles were prepared in the following manner. First, latex particles were added to 10 mM HEPES-NaOH (pH 7.7) so that their concentration would be 0.5 w/v%. To 1 mL of this latex particle dispersion, 0.3 mL of 1 mg/mL WSC aqueous solution was added, and they were inverted at 25°C for 15 minutes. To this inverted dispersion, 0.5 mL of PBS containing 1 mg/mL anti-CMA antibody was added, and they were inverted at 25°C for 60 minutes. To this inverted dispersion, 454 µL of 10 mM HEPES-NaOH (pH 7.7) containing 10 w/v% BSA was further added, and the latex particles were suspended. Then, they were inverted at 25°C for 60 minutes. Subsequently, the dispersion was centrifuged at 15,000 rpm and 10°C for 30 minutes, and the supernatant was removed. To the obtained residue, 3 mL of 10 mM HEPES-NaOH (pH 7.7) containing 0.1% Tween 20 and 0.1% BSA was added, and the latex particles were suspended. Then, the suspension was centrifuged again at 15,000 rpm and 10°C for 30 minutes, and the supernatant was removed. To the obtained residue, 2.5 mL of 20 mM MOPS-NaOH (pH 7.5) containing 5% glycerol, 0.05% BSA, and 0.05% sodium azide was added, and the latex particles were suspended, thus obtaining the anti-CMApolyclonal antibody-sensitized latex particles.

### (Example 1)

### [Pretreatment Process]

As pretreatment of a sample, equal parts of blood serum and a 7M urea aqueous solution were mixed, and they were incubated at 25°C for 30 minutes. The pH of the pretreatment solution was 7.

### [Immunological Measurement Process]

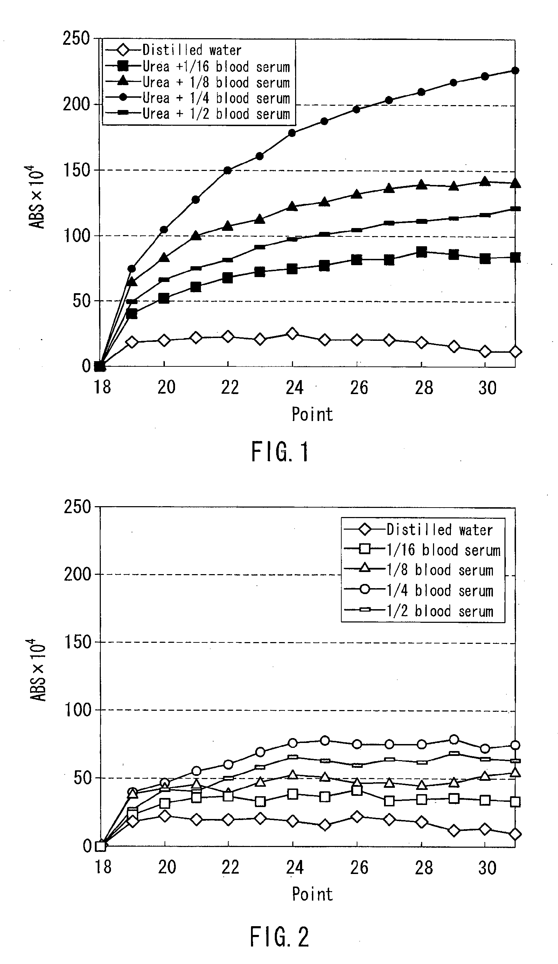

20 µL of the pretreated sample was mixed with 115 µL of first reagent listed below, and the sample was incubated at 37°C for 5 minutes. Then, to the incubated sample, 115 µL of second reagent was added, and the absorbance of the sample at 660 nm was measured to obtain an initial value. Further, the sample was further incubated at 37°C for 5 minutes, and then the absorbance of the sample at 660 nm was measured, and the difference (Δ absorbance) between the obtained measured value and the initial value was obtained. Further, the pretreated sample was diluted with distilled water to prepare 2-fold, 4-fold and 8-fold diluted samples, and they were measured in the same manner as above. FIG. 1 provides the results. The vertical axis indicates Δ absorbance, and the horizontal axis indicates measuring time, where 1 point is equivalent to 19.92 sec. The measurements were carried out using an automatic analyzer (7070, manufactured by Hitachi Ltd).
<First Reagent>
   1% PEG 20000
   1% BSA
   0.9% NaCl
   0.1% EDTA₂Na
   0.09% NaN₃
   0.2M Tris-HCl buffer
   pH 8.4
<Second Reagent>
   0.05% anti-CMA polyclonal antibody-sensitized latex particles (diameter: 370 nm, white-colored)
   5% Glycerol
   0.05% BSA
   0.05% NaN₃
   20mM MOPS
   pH 7.5

### (Comparative Example 1)

As Comparative Example 1, equal parts of blood serum and distilled water were mixed, and the sample was measured in the same manner as in Example 1 except that the sample was not pretreated. FIG. 2 provides the results.

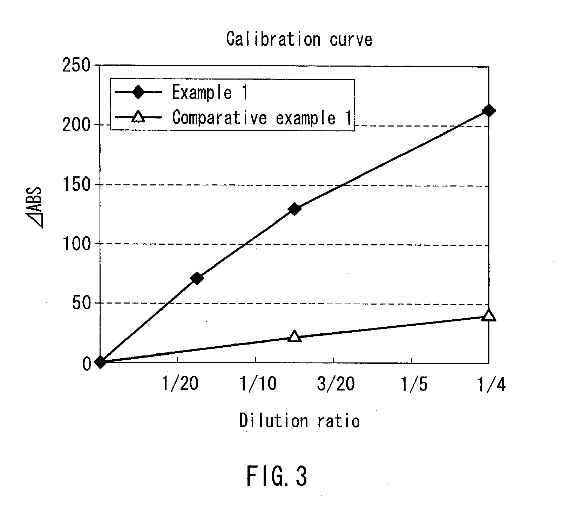

For Example 1 and Comparative Example 1, calibration curves were plotted based on the results shown in FIGS. 1 and 2. FIG. 3 provides the results. The vertical axis indicates Δ absorbance and the horizontal axis indicates the dilution factors of the samples (blood serum). As shown in FIGS. 1 to 3, the samples pretreated with urea showed a significant increase in the reactivity in comparison with those not being pretreated. Further, pretreatment of the samples with urea resulted in an increase in Δ absorbance in a concentration dependent manner. Thus, according to the measuring method of the present invention, it was shown that the concentration of CMA in the biological sample could be measured by an immunological method.

The method of analyzing a sample of the present invention is useful in a variety of fields such as the medical field and the clinical testing field.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A method of measuring carboxymethylarginine in a sample, the method comprising:
treating the sample with a pretreatment agent comprising urea; and
measuring carboxymethylarginine in the treated sample by an immunological method.

2. The method according to claim 1, wherein the pretreatment agent comprises urea as an active ingredient.

3. The method according to claim 1 or 2, wherein the sample is a biological sample.

4. The method according to claim 3, wherein the biological sample is a urine-derived, blood-derived, or tissue-derived sample.

5. The method according to any one of claims 1 to 4, wherein the immunological method is selected from the group consisting of enzyme immunoassay, immunochromatography, and agglutination methods.

6. The method according to any one of claims 1 to 5, wherein the immunological method comprises mixing the treated sample with antibody-sensitized particles sensitized with an antibody reactive to carboxymethylarginine, and measuring agglutination of the antibody-sensitized particles.

7. The method according to claim 6, wherein the antibody-sensitized particles are antibody-sensitized particles obtained by sensitizing insoluble carrier particles to an antibody.

8. The method according to claim 7, wherein the insoluble carrier particles are selected from the group consisting of latex particles and colloidal gold particles.

9. Use of a pretreatment agent comprising urea for treating a sample in measuring carboxymethylarginine by an immunological method.

10. Use according to claim 9, wherein the pretreatment agent comprises an effective amount of urea as an active ingredient.

11. A measuring kit for use in measuring carboxymethylarginine by an immunological method, the measuring kit comprising:
a pretreatment agent comprising urea; and
a measuring reagent for use in measuring carboxymethylarginine by an immunological method.

12. The measuring kit according to claim 11, wherein the measuring reagent comprises antibody-sensitized particles.

13. The measuring kit according to claim 12, wherein the antibody-sensitized particles are antibody-sensitized particles obtained by sensitizing insoluble carrier particles to an antibody.

14. The measuring kit according to claim 13, wherein the insoluble carrier particles are selected from the group consisting of latex particles and colloidal gold particles.

15. A method for pretreating a sample containing carboxymethylarginine, comprising treating the sample with a pretreatment agent including urea.

## Patentansprüche

1. Verfahren zum Messen von Carboxymethylarginin in einer Probe, wobei das Verfahren Folgendes umfasst:
das Behandeln der Probe mit einem Vorbehandlungsmittel, das Harnstoff umfasst; und
das Messen von Carboxymethylarginin in der behandelten Probe durch ein immunologisches Verfahren.

2. Verfahren nach Anspruch 1, wobei das Vorbehandlungsmittel Harnstoff als Wirkstoff umfasst.

3. Verfahren nach Anspruch 1 oder 2, wobei die Probe eine biologische Probe ist.

4. Verfahren nach Anspruch 3, wobei die biologische Probe eine von Urin stammende, von Blut stammende oder von Gewebe stammende Probe ist.

5. Verfahren nach einem der Ansprüche 1 bis 4, wobei das immunologische Verfahren aus der Gruppe ausgewählt ist bestehend aus Enzymimmunoassay-, Immunchromatographie- und Agglutinationsverfahren.

6. Verfahren nach einem der Ansprüche 1 bis 5, wobei das immunologische Verfahren das Mischen der behandelten Probe mit antikörpersensibilisierten Teilchen, die mit einem Antikörper sensibilisiert sind, der auf Carboxymethylarginin reaktiv ist, und das Messen der Agglutinierung der antikörpersensibilisierten Teilchen umfasst.

7. Verfahren nach Anspruch 6, wobei die antikörpersensibilisierten Teilchen antikörpersensibilisierte Teilchen sind, die durch Sensibilisieren unlöslicher Trägerteilchen auf einen Antikörper erhalten werden.

8. Verfahren nach Anspruch 7, wobei die unlöslichen Trägerteilchen aus der Gruppe ausgewählt sind bestehend aus Latexteilchen und kolloidalen Goldteilchen.

9. Verwendung eines Vorbehandlungsmittels, das Harnstoff umfasst, zur Behandlung einer Probe beim Messen von Carboxymethylarginin durch ein immunologisches Verfahren.

10. Verwendung nach Anspruch 9, wobei das Vorbehandlungsmittel eine wirksame Menge Harnstoff als Wirkstoff umfasst.

11. Messkit zur Verwendung beim Messen von Carboxymethylarginin durch ein immunologisches Verfahren, wobei das Messkit Folgendes umfasst:
ein Vorbehandlungsmittel, das Harnstoff umfasst; und
ein Messreagens zur Verwendung beim Messen von Carboxymethylarginin durch ein immunologisches Verfahren.

12. Messkit nach Anspruch 11, wobei das Messreagens antikörpersensibilisierte Teilchen umfasst.

13. Messkit nach Anspruch 12, wobei die antikörpersensibilisierten Teilchen antikörpersensibilisierte Teilchen sind, die durch Sensibilisieren unlöslicher Trägerteilchen für einen Antikörper erhalten werden.

14. Messkit nach Anspruch 13, wobei die unlöslichen Trägerteilchen aus der Gruppe ausgewählt sind bestehend aus Latexteilchen und kolloidalen Goldteilchen.

15. Verfahren zur Vorbehandlung einer Probe, die Carboxymethylarginin enthält, umfassend das Behandeln der Probe mit einem Vorbehandlungsmittel, das Harnstoff enthält.

## Revendications

1. Procédé de mesure de carboxyméthylarginine dans un échantillon, le procédé comprenant :
le traitement de l'échantillon avec un agent de prétraitement comprenant de l'urée ; et
la mesure de carboxyméthylarginine dans l'échantillon traité par un procédé immunologique.

2. Procédé selon la revendication 1, dans lequel l'agent de prétraitement comprend de l'urée comme ingrédient actif.

3. Procédé selon la revendication 1 ou 2, dans lequel l'échantillon est un échantillon biologique.

4. Procédé selon la revendication 3, dans lequel l'échantillon biologique est un échantillon obtenu à partir d'urine, de sang, ou de tissu.

5. Procédé selon l'une quelconque des revendications 1 à 4, dans lequel le procédé immunologique est sélectionné à partir du groupe constitué par un essai immuno-enzymatique, une immunochromatographie, et des procédés d'agglutination.

6. Procédé selon l'une quelconque des revendications 1 à 5, dans lequel le procédé immunologique comprend le mélange de l'échantillon traité avec des particules sensibilisées par un anticorps, sensibilisées par un anticorps réactif à la carboxyméthylarginine, et la mesure de l'agglutination des particules sensibilisées par un anticorps.

7. Procédé selon la revendication 6, dans lequel les particules sensibilisées par un anticorps sont des particules sensibilisées par un anticorps obtenues en sensibilisant des particules porteuses insolubles à un anticorps.

8. Procédé selon la revendication 7, dans lequel les particules porteuses insolubles sont sélectionnées à partir du groupe constitué par des particules de latex et des particules d'or colloïdal.

9. Utilisation d'un agent de prétraitement comprenant de l'urée pour traiter un échantillon en mesurant la carboxyméthylarginine par un procédé immunologique.

10. Utilisation selon la revendication 9, dans laquelle l'agent de prétraitement comprend une quantité efficace d'urée comme ingrédient actif.

11. Kit de mesure à utiliser dans la mesure de carboxyméthylarginine par un procédé immunologique, le kit de mesure comprenant :
un agent de prétraitement comprenant de l'urée ; et
un réactif de mesure à utiliser dans la mesure de carboxyméthylarginine par un procédé immunologique.

12. Kit de mesure selon la revendication 11, dans lequel le réactif de mesure comprend des particules sensibilisées par un anticorps.

13. Kit de mesure selon la revendication 12, dans lequel les particules sensibilisées par un anticorps sont des particules sensibilisées par un anticorps obtenues en sensibilisant des particules porteuses insolubles à un anticorps.

14. Kit de mesure selon la revendication 13, dans lequel les particules porteuses insolubles sont sélectionnées à partir du groupe constitué par des particules de latex et des particules d'or colloïdal.

15. Procédé pour prétraiter un échantillon contenant de la carboxyméthylarginine, comprenant le traitement de l'échantillon avec un agent de prétraitement incluant de l'urée.
